Europäisches Patentamt

European Patent Office

Office européen des brevets

(11) Publication number: **0 272 306**
**B1**

# (12) EUROPEAN PATENT SPECIFICATION

(45) Date of publication of patent specification: **29.08.90**

(21) Application number: **87904444.4**

(22) Date of filing: **11.06.87**

(86) International application number:
**PCT/US87/01443**

(87) International publication number:
**WO 88/00176 14.01.88 Gazette 88/02**

(51) Int. Cl.⁵: **C 07 C 2/02**

(54) OLIGOMERIZATION OF OLEFINS.

(30) Priority: **27.06.86 US 879787**

(43) Date of publication of application:
**29.06.88 Bulletin 88/26**

(45) Publication of the grant of the patent:
**29.08.90 Bulletin 90/35**

(84) Designated Contracting States:
**BE DE FR GB IT NL**

(56) References cited:
**EP-A-0 142 154**
**EP-A-0 205 711**
**WO-A-85/04853**
**US-A-4 531 014**

(73) Proprietor: **MOBIL OIL CORPORATION**
**150 East 42nd Street**
**New York New York 10017 (US)**

(72) Inventor: **KIRKER, Garry, Wayne**
**18 Old Mill Road**
**Sewell, NJ 08080 (US)**
Inventor: **LANDIS, Michael, Eugene**
**26 North Horace Street**
**Woodbury, NJ 08096 (US)**
Inventor: **PAGE, Nancy, Marie**
**289 Flint Court S.**
**Yardley, PA 19067 (US)**

(74) Representative: **Colmer, Stephen Gary**
**Patent Department c/o Mobil Services Company**
**Limited Mobil Court 3 Clements Inn**
**London WC2A 2EB (GB)**

Courier Press, Leamington Spa, England.

**Description**

This invention relates to oligomerization of olefins.

It is known from, for example, US Patent No. 3,960,978 to oligomerize $C_2$—$C_5$ olefins over aluminosilicate zeolites, particularly ZSM-5 and ZSM-11, to produce a gasoline fraction containing predominantly olefinic compounds and not more than about 20% by weight of aromatics. The process involves contacting the olefinic feed with the zeolite at a temperature of 260—482°C, a WHSV of 0.1 to 25 and a hydrocarbon partial pressure of 50 to 4050 kPa.

It is also known from, for example, EP—B—20017, to convert olefinic gasoline containing at least 50% by weight of olefins, mainly $C_6+$ olefins, to fuel oil and gasoline of improved gum stability by contacting the feed with a zeolite having a constraint index of 1—12 and a silica to alumina mole ratio of at least 12, preferably ZSM-5, at a temperature of 177—316°C, a pressure of 790—7900 kPa and a LHSV of 0.2—10.

It has now been found that thermally stable layered chalcogenides containing chalcogenide pillars separating the layers may be employed to oligomerize olefins to produce hydrocarbons boiling in the gasoline to light distillate range (66°—260°C) when the feed comprises $C_3$—$C_5$ olefins, and to produce hydrocarbons boiling in the heavy distillate to lube range (260—566°C) when the feed comprises $C_6$—$C_{20}$ olefins.

Accordingly, the invention resides in a method for oligomerizing olefins which comprises contacting an olefin-containing feed with a catalyst, which contains a thermally stable, non-swellable layered chalcogenide having adjacent layers separated by chalcogenide pillars, at a temperature of 100—500°C and a pressure of 10—20000 kPa.

Preferably the layered chalcogenide is a layered oxide and most preferably a layered titanate or a layered silicate.

Preferably, the pillars comprise a polymeric oxide and most preferably polymeric silica.

Many layered materials are known which have three-dimensional structures which exhibit their strongest chemical bonding in only two dimensions. In such materials, the stronger chemical bonds are formed in two-dimensional planes and a three-dimensional solid is formed by stacking such planes on top of each other, the interactions between the planes being weaker than the chemical bonds holding an individual plane together. The weaker bonds generally arise from interlayer attractions such as Van der Waals forces, electrostaic interactions, and hydrogen bonding. In those situations where the layered structure has electronically neutral sheets interacting with each other solely through Van der Waals forces, a high degree of lubricity is manifested as the planes slide across each other without encountering the energy barriers that arise with strong interlayer bonding. Graphite is an example of such a material. The silicate layers of a number of clay materials are held together by electrostatic attraction provided by ions located between the layers. In addition, hydrogen bonding interactions can occur directly between complementary sites on adjacent layers, or can be provided by interlamellar bridging molecules.

Laminated materials such as clays may be modified to increase their surface area. In particular, the distance between the layers can be increased substantially by absorption of various swelling agents such as water, ethylene glycol, amines and, ketones, which enter the interlamellar space and push the layers apart. However, the interlamellar spaces of such layered materials tend to collapse when the molecules occupying the space are removed by, for example, exposing the clays to high temperatures. Accordingly, such layered materials having enhanced surface area are not suited for use in chemical processes involving even moderately severe conditions.

The extent of interlayer separation can be estimated by using standard techniques such as X-ray diffraction to determine the basal spacing, also known as "repeat distance" or "d-spacing". These values indicate the distance between, for example, the uppermost margin of one layer with the uppermost margin of its adjoining layer. If the layer thickness is known, the interlayer spacing can be determined by subtracting the layer thickness from the basal spacing.

The method of the present invention utilizes an olefin oligomerization catalyst prepared from a layered starting material which contains anionic ion exchange sites having interlayer or interspathic cations associated therewith. Such cations may include hydrogen ion, hydronium ion and alkali metal cation. The starting material is treated with a "propping" agent, conveniently comprising a source of an organic cation such as organoammonium ion, in order to effect an exchange of or addition to the interlayer cations and thereby separate the layers of the starting material. For example, where the interlayer cations are hydrogen or hydronium ions, the source of organic cation, may include a neutral compound such as organic amine which is converted to a cationic analogue during the "propping" treatment. The foregoing treatment results in the formation of a layered material of enhanced interlayer separation depending upon the size of the organic cation introduced. In one embodiment, a series of organic cation exchanges is carried out. For example, an organic cation may be exchanged with an organic cation of greater size, thus increasing the interlayer separation in a step-wise fashion. Preferably, contact of the layered material with the propping agent is conducted in aqueous medium so that water is trapped within the interlayer spaces of the propped species.

After the ion exchange, the organic-"propped" species is treated with a compound capable of conversion, preferably by hydrolysis, to a chalcogenide, preferably a polymeric oxide. The "propped" layered material containing the chalcogenide precursor is then treated to produce chalcogenide pillars

2

EP 0 272 306 B1

separating the layers. Where the treatment involves hydrolysis, this may for example be carried out using water already present in organic-"propped" layered material.

It is preferred that the organic cation deposited between the layers is capable of being removed from the layered material without substantial disturbance or removal of the chalcogenide pillars or their precursor. For example, organic cations such as n-octylammonium may be removed by calcination or chemical oxidation, although preferably by calcination and preferably after the precursor has been converted to the chalcogenide pillars.

The resulting pillared product exhibits high surface area, e.g., greater than 200, 400, or even 600 $m^2/g$, and thermal stability.

The layered materials used in producing the catalyst employed in the present invention are layered chalcogenides, preferably oxides, of elements having an atomic number from 13 to 15, 21 to 33, 39 to 51, 57 to 83 or greater than 90. The layered oxide is "non-swellable" which is intended to distinguish from conventional clays which contain octahedrally coordinated metal oxide sheets bonded to tetrahedrally coordinated silica sheets and which undergo substantial swelling, sometimes by an essentially unbounded amount, when contacted with water. As used herein in relation to a layered oxide material, the term "non-swellable" is defined as meaning a layered oxide material which, when contacted with at least 10 grams of water per gram of the layered oxide at 23°C for 24 hours, exhibits an increase in d-spacing no greater than 5 Angstrom as compared with the anhydrous material. Included among these materials are $H_2Ti_3O_7$, $Na_2Ti_3O_7$ and $KTiNbO_5$ as well as certain layered perovskites, titanometallates and silicates, for example, the metasilicates magadiite, natrosilite, kenyaite, makatite and kanemite. With certain layered starting materials, for example layered silicates, it has been found to be preferable to treat the silicate with one or more polar solvents prior to or during exchange with the source of organic cation. The polar solvent used should exhibit electric dipole moments in the gas phase of at least 3.0 Debyes (D), preferably at least 3.5 Debyes, most preferably at least about 3.8 D. Examples of suitable solvents are water, dimethylsulfoxide (DMSO) and dimethylformamide (DMF). A table of selected organic compounds and their electric dipole moments can be found in CRC Handbook of Chemistry and Physics, 61st dition, 1980—1981 at pages E-64 to E-66. It is believed that the treatment of the oxide starting material with one or more highly polar solvents facilitates the introduction of the source of organic cation between the layers of the starting material.

In one preferred embodiment, the starting material is a layered oxide of Group IVA metal such as titanium, zirconium and hafnium, with a layered titanate, e.g., a trititanate such as $Na_2Ti_3O_7$, being particularly preferred. Trititanates are commercially available materials whose structure consists of anionic sheets of titanium octahedra with interlayer alkali metal cations. A method for making such material may be found in U.S. Patent 2,496,993. In another preferred embodiment the starting material is a layered silicate having the structure of magadiite.

As previously stated, the layered starting material is treated with an organic compound capable of forming cationic species such as organophosphonium or organoammonium ion, before introduction of the pillar material. Insertion of the organic cation between the adjoining layers serves to physically separate the layers in such a way as to make the layered oxide receptive to the interlayer addition of an electrically neutral, hydrolyzable, chalcogenide precursor. In particular, alkylammonium cations have been found useful in the present invention. Thus $C_3$ and larger alkylammonium, e.g., n-octylammonium, cations are readily incorporated within the interlayer species of the layered oxides, serving to prop open the layers in such a way as to allow incorporation of the polymeric oxide precursor. The extent of the interlayer spacing can be controlled by the size of the organoammonium ion employed so that, with a trititanate as the layered oxide starting material, use of the n-propylammonium cation will achieve an interlayer spacing of 0.2—0.5 nm, whereas to achieve an interlayer spacing of 1.0—2.0 nm an n-octylammonium cation or a cation of equivalent length is required. Indeed, the size and shape of the organic cation can affect whether or not it can be incorporated within the layered oxide structure at all. For example, bulky cations such as tetrapropylammonium are generally undesirable for use in the present method, whereas n-alkyl ammonium cations, such as those derived from n-alkyl primary amines and $R_3R'N^+$ cations, where R is methyl or ethyl and R' is an alkyl group with at least 5 carbon atoms, are preferred. The organic ammonium cations separating the oxide layers may be formed in situ by reaction of the neutral amine species with interlayer hydrogen or hydronium cations of the layered starting material. Alternatively where the interlayer cations of the layered starting material are alkali metal cations, the organic ammonium cation may be formed by initially combining an amine and an aqueous acid solution, such as hydrochloric acid, and then treating the layered oxide with the resulting aqueous organoammonium ion solution. In either case, the treatment is conducted in aqueous media so that water is then available to hydrolyze the electrically neutral, hydrolyzable polymeric oxide precursor subsequently introduced into the "propped" product.

The chalcogenide pillars formed between the layers of the oxide starting material preferably comprise an oxide, and more preferably comprise a polymeric oxide, of at least one element selected from Groups IB, IIB, IIIA, IIIB, IVA, IVB, VA, VB, VIA, VIIA, and VIIIA of the Periodic Table of Elements (Fisher Scientific Co., Cat. No. 5-702-10, 1978). In one preferred embodiment, the pillars comprise an oxide of zirconium, titanium or more preferably of at least one element selected from Group IVB of the Periodic Table, other than carbon, and most preferably include polymeric silica. Where the layered starting material is a silicate, such

3

as magadiite, the pillars preferably comprise polymeric silica and alumina. The polymeric oxide pillars are formed from a precursor material which is preferably introduced between the layers of the organic propped species as a cationic, or more preferably electrically neutral, hydrolyzable compound of the desired element(s).

The precursor material is preferably an organometallic compound which is a liquid under ambient conditions. Suitable polymeric silica precursor materials include tetrapropylorthosilicate, tetramethylorthosilicate and, most preferably, tetraethylorthosilicate. Where the pillars are also required to include polymeric alumina a hydrolyzable aluminum compound can be contacted with the organic "propped" species before, after or simultaneously with the contacting of the layered starting material with the silicon compound. Preferably, the hydrolyzable aluminum compound employed is an aluminum alkoxide, e.g., aluminum isopropoxide.

After hydrolysis to produce the polymeric oxide pillars and calcination to remove the organic propping agent, the final pillared product may contain residual exchangeable cations. For example, sodium titanate pillared with polymeric silica may contain 2—3% by weight of residual sodium. Such residual cations can be ion exchanged by methods well known with other cationic species to provide or alter the catalytic activity of the pillared product. Suitable replacement cations include cesium, cobalt, nickel, copper, zinc, manganese, platinum, lanthanum, aluminum and mixtures thereof.

In one preferred embodiment, where the layered starting material is a titanate and the pillars are formed of silica, the resulting silicotitanate product exhibits the characteristic X-ray diffraction pattern shown in Table 1 below.

TABLE 1
Composite list of principal X-ray powder*
Diffraction peaks for silicotitanates

| Line number | (2 theta—2 theta) (minimum maximum) | 100 I/Io (relative intensity) range |
|---|---|---|
| 1 | less than or equal to 8.7 | VS to W |
| 2 | 11.1—14.3 | S to W |
| 3 | 11.8—15.2 | M to W |
| 4 | 24.5—25.0 | VS to W |
| 5 | 25.0—25.4 | M to W |
| 6 | 28.5—30.2 | VS to W |
| 7 | 29.8—30.6 | S to W |
| 8 | 33.0—33.5 | S to W |
| 9 | 43.2—43.5 | M to W |
| 10 | 44.2—44.7 | M to W |
| 11 | 48.5—48.9 | VS to M |
| 12 | 52.7—52.9 | W |

*2 Theta minimum—2 Theta maximum=Range of 2 Theta-values observed for eight specific pillared silicotitanates.

These values were determined by standard techniques. The radiation was the K-alpha doublet of copper, and a scintillation counter spectrometer was used. The peak heights, I, and the positions as a function of 2 times theta, where theta is the Bragg angle, were determined. From these, the relative intensities, $I/I_o$ where $I_o$ is the intensity of the strongest line or peak, and d is the interplanar spacing in angstroms (A), corresponding to the recorded lines, were calculated. The relative intensity in the table above is expressed as follows:

| Relative intensity | 100 I/Io |
|---|---|
| VS (Very Strong) | 60—100 |
| S (Strong) | 40—60 |
| M (Medium) | 20—40 |
| W (Weak) | 0—20 |

Variations in the interplanar spacing and relative intensity may occur as a result of ion exchange, changes in the composition of the silicotitanate, or exposure to calcination conditions:

Prior to use as a catalyst in the present oligomerization method, pillared titanate materials containing an exchangeable cation between the layers such as an alkali metal ion, e.g. sodium ion, may be treated by exchange with 1) ammonium ion or 2) hydrogen ion and/or 3) calcination to form a proton-exchanged material which may be at least partially exchanged with replacement cations such as cesium, cerium, cobalt, nickel, copper, zinc, manganese, platinum, lanthanum, aluminum, ammonium, hydronium and mixtures thereof. Ni(II) and Al(III) cations are of particularly significant interest as replacement cations. Layered titanates prepared without appreciable alkali metal content, e.g. by exchanging out the alkali metal with acid treatment, are also particularly well suited to such oligomerizations. Such materials have an alkali metal content below about 1.0 weight percent, preferably below about 0.5 weight percent.

The layered silicates employed in the present invention are preferably high silica alkali silicates whose layers lack octahedral sheets and which are prepared hydrothermally from aqueous reaction mixture containing silica and caustic at relatively moderate temperatures and pressures. The layered silicates may contain tetracoordinate framework atoms other than Si in the layers which can be introduced by co-crystallizing in the presence of non-silicon tetravalent elements, e.g. those selected from the group consisting of Al, B, Co, Cr, Fe, Ga, In, Ni, Zr as well as any other such elements which are catalytically useful when incorporated in the silicate structure. Alternatively, non-silicon framework elements already in a layered silicate may be substituted by a different tetracoordinate element. For example, kenyaite containing boron in its framework when treated with aluminum nitrate results in a kenyaite which contains aluminum in its framework. Both co-crystallized and substituted layered high silica alkali silicates may be treated to provide layered materials containing interspathic oxide pillars.

The preferred layered silicate is synthetic substituted magadiite. Synthetic magadiite is readily produced hydrothermally from a reaction mixture containing inexpensive sources of silica and caustic. Tetracoordinate elements X other than silicon, e.g., Al, B, Co, Cr, Fe, Ga, In, Ni, Zr, preferably Al or Fe, may be added to the reaction mixture as may a suitable organic directing agent R. The reaction mixture for such synthetic magadiite-type materials can be described in molar ratios as follows:

$SiO_2/X_2O_3 = 10$ to infinity where X can be Al, B, Co, Cr, Fe, Ga, and/or Ni or other catalytically useful metal
$M^+OH^-/SiO_2 = 0$ to 0.6, (preferably 0.1—0.6) M = any alkali metal
$H_2O/SiO_2 = 8$—500
$R/SiO_2 = 0$—0.4

where R can be an organic such as benzyltriethylammonium chloride, benzyltrimethylammonium chloride, dibenzyldimethylammonium chloride, N,N—dimehylpiperazine, triethylamine, or other quaternary compounds or heterocyclic amines.

The reaction mixture is maintained at a temperature of 100 to 200°C for anywhere from 1 to 150 days in order to form a product having the following composition:

% N = 0—3, e.g., 0 to 0.3
$SiO_2/X_2O_3 = 10$ to infinity where X is in the tetrahedral or octahedral position
$M_2O/SiO_2 = 0$ to 0.5, e.g., 0.05—0.1

The synthetic layered silicate material thus prepared is of low surface area. Introduction of interspathic polymeric oxides according to the method of the present invention can increase the surface area of the material. Generally, the synthetic magadiite-type material is acidified by any suitable means, e.g., treatment with aqueous 0.1 N HCl, before being treated with a "propping" agent, alone or combined with a suitable polar solvent.

Where the method of the invention is employed to effect oligomerization of low molecular weight olefins ($C_3$—$C_5$) hydrocarbons boiling in the gasoline or light distillate range (66 to 260°C or preferably 93 to 232°C), the conversion is conveniently carried out in the vapor-phase by contacting the olefin feed in a reaction zone, such as, for example, a fixed bed of catalyst composition, under conversion effective conditions including a temperature of 150 to 430°C, preferably 260 to 400°C, and a pressure of 100—10000 kPa preferably 100—3200 kPa. The weight hourly space velocity (WHSV) may be maintained at 0.2 hr$^{-1}$ to 20 hr$^{-1}$, preferably from 0.5 hr$^{-1}$ to 4 hr$^{-1}$. In addition water can be co-fed with the olefin feedstock, preferably in an amount ranging from 0 to 5 moles of water/mole of olefin feedstock. Within these limits the conditions of temperature and pressure will vary considerably depending upon equilibrium considerations, exact feed material, and presence or absence of diluents, such as, for example, $C_1$—$C_4$ paraffins, such as methane, ethane, propane, isobutane and n-butane; and hydrogen sulfide. The amount of diluent which may be present may vary between 0 and 90 preferably 20 to 60, weight percent based on the weight of olefin feedstock. This process may be conducted in either batch or fluid bed operation with attendant benefits of either operation readily obtainable.

The method of the invention may also be used to oligomerize intermediate molecular weight olefins, $C_6$ to $C_{20}$, preferably $C_{10}$ to $C_{16}$, in order to form hydrocarbons boiling in the heavy distillate to lube range (260 to 566°C, preferably 316 to 454°C) i.e., $C_{20}^+$ hydrocarbons. Process conditions in a sealed reactor can be adjusted to favor the formation of $C_{20}^+$ materials by using moderate reaction temperatures of 140 to 285°C depending on the olefin feed used, at autogenous pressures during contact with the layered titanate composition. When feeds of 1-decene are employed the temperature preferably ranges from 140 to 160°C.

When feeds of 1-hexadecene are used, reaction temperatures of 265 to 285°C are preferred. Oligomerization conditions which may be applied to the oligomerization of intermediate molecular weight olefins to many distillates and lubes may be found in U.S. Patent No. 4,542,247.

The present invention is illustrated further by the following examples. In these examples, when alpha value is reported, it is noted that the alpha value is an approximate indication of the catalytic cracking activity of the test catalyst compared to a standard catalyst and gives the relative rate constant (rate of normal hexane conversion per volume of catalyst per unit time) compared to the activity of the highly active silica alumina cracking catalyst taken as an alpha of 1 (Rate Constant=0.16 sec$^{-1}$). The alpha test is described in U.S. Patent 3,354,078 and in *The Journal of Catalysis*, Vol. IV. pp. 522—529 (August 1965).

Example 1

A mixture of 500 g $Na_2Ti_3O_7$, 427 g n-octylamine, 309.7 g of 37.8% HCl and 7000 g water was refluxed for 22 hours. The resultant solution was decanted, filtered and then dried on the filter at room temperature overnight. This product was then treated twice with absolute ethanol and water as follows: the solid product was reslurried in 2 liters ethanol, filtered, and air-dried 6 hours at room temperature. This material was then slurried in 1.5 liters water, heated at 100°C in a 2 liter polypropylene jar for 17 hours, filtered and dried at room temperature for 24 hours.

450 g of the dried product were mechanically stirred in 3000 g of tetraethylorthosilicate in a 10 liter beaker covered with perforated aluminum foil for 68 hours at room temperature and then filtered and dried in air at room temperature for about 4 days. This material was calcined in nitrogen at 510°C (950°F) for 2 hours and then in air for one hour at 510°C (950°F). The silicotitanate product had a surface area of 405 m$^2$/g and the following composition (wt.%):

| | |
|---|---|
| $TiO_2$ | 51.7 |
| $SiO_2$ | 39.9 |
| Na | 1.8 |
| Ash | 98.1 |

This material had an alpha-value of 3.

A 5.0 gm sample of the pillared silicotitanate material was charged to a 450 cc autoclave, together with about 150 cc liquid propylene and the temperature was raised to 150°C while the pressure quickly rose to 7272 kPa (1040 psig). The reaction was allowed to continue for a total of 24 hours at a stirring rate of 1000 rpm. At the end of this time, unreacted propylene was vented from the autoclave and a total of 3.4 grams of liquid product were recovered. Gas chromatographic analysis of this liquid product showed it to have the following product distribution (wt.%):

| Component | Wt.% |
|---|---|
| $C_6$ | 25.2 |
| $C_9$ | 53.5 |
| $C_{12}$ | 16.7 |
| $C_{15}$ | 4.3 |
| $C_{18}$ | 0.3 |
| $C_{21}+$ | 0 |

Example 2

A mixture of 500 g $Na_2Ti_3O_7$, 770 g n-octylamine, 559 g of 37.8% HCl and 5 liters of water was refluxed for 22 hours. The solution was cooled to 70°C and 281 g of 37.8% HCl was added. The product was filtered, washed with 10 l of hot water and dried overnight at room temperature. The solid product was slurried in 3 liters of ethanol for one hour at room temperature, filtered and air dried 24 hours at room temperature. 900 Grams of this material were slurried in 4 liters of water and stirred at room temperature for 23 hours, filtered and dried at room temperature for 24 hours. 825 g of the dried product were mechanically stirred in 5.5 kg of tetraethylorthosilicate in a 10 liter beaker covered with perforated aluminum foil for 72 hours at room temperature and then filtered and dried in air at room temperature for about 24 hours. This material was calcined in nitrogen at 510°C (950°F) for 2 hours and then in air for one hour at 510°C (950°F). The silicotitanate product had a surface area of 299 m$^2$/g and the following composition (wt.%):

| | |
|---|---|
| $TiO_2$ | 70.2 |
| $SiO_2$ | 21.7 |
| Na | 3.3 |
| Ash | 100.0 |

7.5 Grams of the pillared silicotitanate product were nickel-exchanged by addition to a 0.5 M $Ni(NO_3)_2$ solution prepared by dissolving 72.5 g $Ni(NO_3)_2 \cdot 6H_2O$ in 500 ml deionized water in a 1 liter flask. The resulting solution was stirred at room temperature for about 68 hours. The catalyst was then filtered from the solution, washed with de-ionized water, and dried for one hour in an oven maintained at 100°C. The catalyst was then pelletized and sized to 1.2—0.68 mm (14/25 mesh). The pelletized catalyst was then carefully calcined by heating from ambient temperature to 400°C at the rate of 1.5°C/min, then held at 400°C for 4 hours.

5.0 Grams of this catalyst was charged to a 300 cc autoclave. Approximately 150 ml liquid propylene was added. The temperature was adjusted to 150°C. Pressure in the autoclave rapidly increased to 10030 kPa (1440 psig). The reaction was allowed to continue for a total of 24 hours. At this time, unreacted propylene was vented from the autoclave. Approximately 8.8 grams liquid product were recovered from the reactor. Gas chromatographic analysis of this material showed it to have the following composition:

| Component | Wt.% |
|---|---|
| $C_6$ | 16.6 |
| $C_9$ | 56.7 |
| $C_{12}$ | 17.7 |
| $C_{15}$ | 6.7 |
| $C_{18}$ | 2.0 |
| $C_{21}+$ | 0.4 |

Example 3

A 1.0 M solution of $NH_4NO_3$ was prepared by dissolving 80 gm $NH_4NO_3$ in one liter de-ionized water in a 2 liter flask. 15 gm of the pillared silicotitanate material of Example 1 were added and the mixture stirred at room temperature for approximately 70 hours. The catalyst was filtered from the solution, washed thoroughly with de-ionized water and dried for one hour in an oven maintained at 100°C. 7 Grams of this catalyst were further treated by heating from ambient temperature to 250°C at the rate of 1°C/min, then held at 250°C for a total of four hours. 5.0 Grams of this catlayst were charged to a 300 cc autoclave. Approximately 150 cc liquid propylene were added and the temperature adjusted to 150°C. The pressure quickly rose to 9930 kPa (1425 psig). The reaction was allowed to continue with stirring at 1000 rpm for a total of 22.5 hours. At the end of this period, unreacted propylene was vented from the autoclave and approximately 3.7 grams of liquid product were recovered. Gas chromatographic analysis of this material showed it to have the following composition

| Component | Wt.% |
|---|---|
| $C_6$ | 22.1 |
| $C_9$ | 60.3 |
| $C_{12}$ | 14.1 |
| $C_{15}$ | 3.4 |
| $C_{18}$ | 0.1 |
| $C_{21}+$ | 0 |

Another 7 grams of this catalyst were calcined by heating from ambient temperature to 500°C at 2°C/min, then held at this temperature for a total of 12 hours. 5 Grams of the resulting catalyst were charged to a 300 cc autoclave, 150 cc liquid propylene were added and the reaction temperature increased to 150°C. The pressure reached 9860 kPa (1415 psig). The reaction was allowed to continue for a total of 24 hours under autogenous pressure at a stirring rate of 1000 rpm. At the end of this time, unreacted propylene was vented fron the autoclave and a total of 4.6 grams of liquid product were recovered. GC analysis of this material showed it to have the following product distributions:

| Component | Wt.% |
|---|---|
| $C_6$ | 15.4 |
| $C_9$ | 60.1 |
| $C_{12}$ | 18.6 |
| $C_{15}$ | 4.9 |
| $C_{18}$ | 1.1 |
| $C_{21}+$ | 0 |

Example 4

300 g of the silicotitanate product of Example 2 (non-nickel-exchanged) were exchanged in triplicate with 3 liters of 0.5 M $Al(NO_3)_3 \cdot 9H_2O$ at room temperature with stirring for 24 hours. The sample was filtered, washed with 3 liters of water, and dried at 120°C for 1 hour after the first and second exchanges. After the third exchange, the product was filtered, washed with 10 liters water, dried at 120°C for 1 hour and calcined in air at 427°C (800°F) for 1 hour. This product had a surface area of 223 m²/g and the following composition (wt.%):

| | |
|---|---|
| $TiO_2$ | 66.7 |
| $SiO_2$ | 23.6 |
| Na | 2.5 |
| $Al_2O_3$ | 0.65 |
| Ash | 97.96 |

5.0 Grams of this catalyst were loaded into a 300 cc autoclave. Approximately 150 cc of liquid propylene were added and the reaction temperature adjusted to 150°C. The pressure rose to 9860 kPa (1415 psig). The reaction was allowed to continue with stirring at 1000 rpm for a total of 24 hours. At the end of this time, the unreacted propylene was vented from the reactor and a total of 14.5 gms liquid product were recovered. Gas chromatographic analysis of this material showed it to contain the following composition:

| Component | Wt.% |
|---|---|
| $C_6$ | 13.3 |
| $C_9$ | 52.3 |
| $C_{12}$ | 24.4 |
| $C_{15}$ | 8.0 |
| $C_{18}$ | 2.0 |
| $C_{21}+$ | 0 |

Example 5

1 kg of $Na_2Ti_3O_7$ was exchanged in triplicate with 16 liters of 1.0 M HCl with stirring at 77°C (170°F) for 24 hours in order to exchange out alkali metal ions. The solid was filtered and washed with 4 liters of water after the first two exchanges. After the third exchange, the product was filtered, washed chloride-free with water, and dried at 77°C (170°F) for 1 hour *in vacuo*. A mixture of 700 g of this material in 700 g n-octylamine and 10.5 liters water was refluxed with stirring for 23 hours. The product was filtered, washed with 10 liters of hot water, and dried at room temperature for 3 days. 600 g of this product were stirred in 4 kg tetraethylorthosilicate for 67 hours at room temperature, filtered and dried for 24 hours at room temperature. 800 Grams of the dried product were calcined in $N_2$ at 510°C (950°F) for 2 hours and in air for 1 hour at 510°C (950°F). The final silicotitanate product had a surface area of 394 $M^2/g$ and the following composition (wt.%):

| | |
|---|---|
| $TiO_2$ | 65.2 |
| $SiO_2$ | 37.3 |
| Na | 0.34 |
| Ash | 97.61 |

5.0 Grams of the catalyst were charged to a 300 cc autoclave. Approximately 150 cc liquid propylene were charged and the temperature adjusted to 150°C. The pressure increased to 9580 kPa (1375 psig). The reaction was allowed to continue with stirring at 1000 rpm for a total of 23.5 hours under autogenous pressure. At the end of this time, unreacted propylene was vented from the autoclave and approximately 69.3 grams liquid product were recovered. Gas chromatographic analysis of this material showed it to have the following composition (wt.%):

| Component | Wt.% |
|---|---|
| $C_6$ | 4.2 |
| $C_9$ | 48.7 |
| $C_{12}$ | 36.5 |
| $C_{15}$ | 10.5 |
| $C_{18}$ | 0.1 |
| $C_{21}+$ | 0 |

Example 6 (Comparative)

10 Grams of a dense form $Na_2Ti_3O_7$ (purchased from Alfa Products) were exchanged with $Ni(NO_3)_2 \cdot 6H_2O$ using the same procedure described in Example 2. 5 Grams of the resulting catalyst were charged to a 300 cc autoclave, approximately 150 ml liquid propylene added and the temperature increased to 150°C. Initial pressure was 10445 kPa (1500 psig). After 16 hours at these conditions, no pressure drop had been observed. At this point the reaction was terminated. This result demonstrates that pillaring is necessary for the catalytic activity of these catalysts.

Example 7

5 Grams of the catalyst prepared in Example 5 were charged to a 300 cc autoclave along with 75 grams

8

of 1-hexadecene that had been percolated over alumina just prior to use. The autoclave was heated to 275°C and the reaction allowed to continue in the liquid phase for a total of 68 hours with stirring at 950 rpm. At the end of this time, the reactor was opened and the products analyzed by gas chromatography. The recovered liquid was found to contain 18.7% (by weight) of the lube range dimer, $C_{32}$. The unreacted monomer was distilled from the dimer. The VI of this $C_{32}$ material was determined to be 138 with a kinematic viscosity of 4.5 cSt at 100°C.

Example 8

5 Grams of the catalyst prepared in Example 5 were charged to a 300 cc autoclave along with 75 grams 1-decene that had been percolated over alumina just prior to use. The temperature in the autoclave was raised to 150°C and the reaction allowed to continue under these conditions with stirring at 1000 rpm for a total of 28 hours. A sample of the liquid after this time showed conversion of 1-decene to be 26.1% (by weight). The observed oligomer selectivities were as follows: $C_{20}=88.1\%$, $C_{30}=10.5\%$, $C_{40}=1.4\%$. After this time, the temperature was increased to 200°C and the reaction allowed to continue for an additional 118 hours at this temperature. At the end of this time, the reaction was terminated. Analysis of the liquid product showed conversion of 1-decene to be 65.5%. The selectivity to the various oligomers was as follows: $C_{20}=83.7\%$, $C_{30}=14.8\%$, $C_{40}=1.6\%$.

The liquid from this reaction was distilled to remove any unreacted 1-decene and a significant portion of the dimeric products. The resulting liquid product had the following product distribution: $C_{20}=27.2\%$, $C_{30}=62.3\%$, $C_{40}=10.4\%$. The V.I. of this material was measured to be 94.3 with a kinematic viscosity of 4.9 cSt at 100°C.

Example 9

5 Grams of the catalyst of Example 2 were charged to a 300 cc autoclave along with 75 grams of 1-hexadecene that had been percolated over activated alumina just prior to use. The temperature was increased to 275°C and the reaction allowed to continue for 16 hours. Analysis of the liquid after this time showed conversion to be 14.7%. The temperature was raised to 325°C and the reaction allowed to continue for an additional 100 hours. After this time, the reaction was terminated. Analysis of the liquid showed 14% conversion to the dimer, $C_{32}$. Unreacted monomer was then distilled from the $C_{32}$ product. The VI of the lube range material was determined to be 115 with a kinematic viscosity of 4.2 cSt at 100°C.

Example 10

An aqueous solution of NaOH was prepared by dissolving 200 g NaOH (98.6% purity) in 8 liters of distilled water. 1350 g of HiSil 233 (at 98.8% solids) was added to this solution to form a slurry (pH 12.4) which was vigorously slurried for 40 minutes. The slurry was then crystallized for 70.5 hours at 150°C (300°F) under autogenous pressure, filtered, washed with in excess of 20 liters of distilled water and, air dried. The resultant magadiite was then exchanged with 0.5 N HCl at pH 2.0 for 24 hours at room temperature, filtered and air dried. A 500 g sample of the acid exchanged magadiite was treated with a solution of octylamine (530 g; Aldrich Chemical Co.) in distilled water (109.7 L) for 24 hours at room temperature. The slurry was filtered on Buchner funnels and washed with about 10 liters of distilled water, then vacuum oven dried for 12 hours at 88°C. A 120 g sample of the dried product was ground to a fine powder then contacted with a solution containing 19 g aluminum isopropoxide (AIP) ALFA Inorganics) dissolved in 747 g of tetraethylorthosilicate (TEOS). The AIP dissolved in TEOS had been heated for 3 hours at 100°C prior to contacting with the powder. After 64 hours contact at room temperature, the product was filtered, air dried and calcined at 538°C (1 hour in $N_2$, 2 hours in air). The properties of the pillared product are summarized in Table 2.

TABLE 2

| S. A., $m^2/g$ | 497 | Elemental analysis |
| --- | --- | --- |
| Sorptions | | C, wt.% 0.05 |
| $H_2O$, g/100 g | 20.0 | Na, ppm 50 |
| Cy-$C_6$ | 14.4 | $SiO_2$, wt.% 93.4 |
| n-$C_6$ | 14.7 | $Al_2O_3$ 2.9 |
| (1000°F) | 5 | Ash 97.3 |
| | | $SiO_2/Al_2O_3=55$ |

XRD Basal Spacing 31.5 A (approx. 20 A opening).

5 g of the resultant catalyst were charged to a 450 cc autoclave along with 75 grams of 1-hexadecene that had been percolated over activated alumina just prior to use. The temperature was increased to 275°C and the reaction allowed to continue with stirring under these conditions for a total of 68 hours. At the end of this time, the liquid was recovered from the autoclave. Gas chromatographic analysis showed the magadiite catalyst had converted 57% of the hexadecene to product. Of this product, 49% of the total product was the dimer, $C_{32}$. The remaining 51% of the liquid product was lighter materials produced presumably by cracking activity of the magadiite. This liquid product was distilled to isolate the lubricant range material. The resulting material had a V.I. of 98.2 with a kinematic viscosity of 8.6 cst at 100°C.

Example 11

A further 5 g of the catalyst of Example 10 were charged to a 450 cc autoclave along with 75 grams of 1-hexadecene that had been percolated over activated alumina just prior to use. The temperature was increased to 200°C and the reaction allowed to continue with stirring under these conditions for a total of 90 hours. At the end of this time, the liquid was recovered from the autoclave. Gas chromatographic analysis showed that 57% of the 1-hexadecene had been converted to dimer. The liquid product was distilled to isolate the dimer. The resulting material had a V.I. of 125.8 with a kinematic viscosity of 6.6 cst at 100°C.

Example 12

5 g of the catalyst of Example 10 were charged to a 450 cc autoclave along with approximately 150 cc liquid propylene. The reaction temperature was increased to 150°C and the pressure in the reaction quickly rose to 7168 kPa (1025 psig). The reaction mixture was stirred at 1000 rpm and the reaction allowed to continue at 150°C and autogenous pressure for a total of 24 hours. At the end of this time, the unreacted propylene was vented from the autoclave. Gas chromatographic analysis of this material showed it to have the following composition:

| Component | Wt.% |
|---|---|
| $C_6$ | 5.6 |
| $C_9$ | 66.8 |
| $C_{12}$ | 22.4 |
| $C_{15}$ | 4.3 |
| $C_{18}$ | 0.9 |
| $C_{21}$ | 0 |

**Claims**

1. A method for oligomerizing olefins comprising contacting an olefin-containing feed with a catalyst which contains a thermally stable, non-swellable layered chalcogenide having adjacent layers separated by chalcogenide pillars at a temperature of 100—500°C and a pressure of 10—20000 kPa.

2. A method as claimed in claim 1 wherein the layered chalcogenide is a layered oxide.

3. A method as claimed in claim 1 wherein the layered chalcogenide is a layered titanate.

4. A method as claimed in claim 3 wherein the pillars comprise a polymeric oxide.

5. A method as claimed in claim 3 wherein the pillars comprise silica.

6. A method as claimed in claim 1 wherein the layered chalcogenide is a layered silicate.

7. A method as claimed in claim 6 wherein the pillars comprise silica and alumina.

8. A method as claimed in claim 1 wherein the catalyst contains metal cations selected from cesium, cerium, cobalt, nickel, copper, zinc, manganese, platinum, lanthanum and aluminum.

9. A method as claimed in any preceding claim wherein the feed contains $C_3$—$C_5$ olefins and is converted to hydrocarbons boiling in the gasoline or light distillate boiling range at a temperature of 150—400°C and a pressure of 100—10000 kPa.

10. A method as claimed in any one of claims 1 to 6 wherein the feed contains $C_6$—$C_{20}$ olefins and is converted to hydrocarbons boiling in the heavy distillate to lube range at a temperature of 140—285°C.

**Patentansprüche**

1. Verfahren zur Oligomerisierung von Olefinen, bei dem eine olefinhaltige Zufuhr mit einem Katalysator bei einer Temperatur von 100 bis 500°C und einem Druck von 10 bis 20000 kPa in Kontakt gebracht wird, der ein thermisch stabiles, nicht quellbares geschichtetes Chalkogenid mit benachbarten Schichten enthält, die durch Chalkogenidstützen getrennt werden.

2. Verfahren nach Anspruch 1, worin das geschichtete Chalkogenid ein geschichtetes Oxid ist.

3. Verfahren nach Anspruch 1, worin das geschichtete Chalkogenid ein geschichtetes Titanat ist.

4. Verfahren nach Anspruch 3, worin die Stützen ein polymeres Oxid umfassen.

5. Verfahren nach Anspruch 3, worin die Stützen Siliciumdioxid umfassen.

6. Verfahren nach Anspruch 1, worin das geschichtete Chalkogenid ein geschichtetes Silicat ist.

7. Verfahren nach Anspruch 6, worin die Stützen Siliciumdioxid und Aluminiumoxid umfassen.

8. Verfahren nach Anspruch 1, worin der Katalysator Metallkationen enthält, die aus Cäsium, Cer, Cobalt, Nickel, Kupfer, Zink, Mangan, Platin, Lanthan und Aluminium ausgewählt sind.

9. Verfahren nach einem der vorstehenden Ansprüche, worin die Zufuhr $C_3$—$C_5$-Olefine enthält und bei einer Temperatur von 150 bis 400°C und einem Druck von 100 bis 10000 kPa in Kohlenwasserstoffe umgewandelt wird, die im Benzinbereich oder im Siedebereich eines leichten Destillats sieden.

# EP 0 272 306 B1

10. Verfahren nach einem der Ansprüche 1 bis 6, worin die Zufuhr $C_6$—$C_{20}$-Olefine enthält und bei einer Temperatur von 140 bis 285°C in Kohlenwasserstoffe umgewandelt wird, die im schweren Destillatbereich bis zum Schmierölbereich sieden.

**Revendications**

1. Un procédé d'oligomérisation d'oléfines consistant à mettre une charge contenant des oléfines au contact d'un catalyseur qui contient un chalcogénure stratifié non susceptible de gonflement et stable à la chaleur, présentant des couches adjacentes séparées par des piliers de chalcogénure, à une température de 100 à 500°C et à une pression de 10 à 20000 kPa.

2. Un procédé selon la revendication 1, dans lequel le chalcogénure stratifié est un oxyde stratifié.

3. Un procédé selon la revendication 1, dans lequel le chalcogénure stratifié est un titanate stratifié.

4. Un procédé selon la revendication 3, dans lequel les piliers contiennent un oxyde polymère.

5. Un procédé selon la revendication 3, dans lequel les piliers contiennent de la silice.

6. Un procédé selon la revendication 1, dans lequel le chalcogénure stratifié est un silicate stratifié.

7. Un procédé selon la revendication 6, dans lequel les piliers contiennent de la silice et de l'alumine.

8. Un procédé selon la revendication 1, dans lequel le catalyseur contient des cations métalliques choisis parmi césium, cérium, cobalt, nickel, cuivre, zinc, manganèse, platine, lanthane et aluminium.

9. Un procédé selon l'une quelconque des revendications précédentes, dans lequel la charge contient des oléfines en $C_3$ à $C_5$ et elle est transformée en hydrocarbures bouillant dans l'intervalle des essences ou des distillats légers à une température de 150 à 400°C et à une pression de 100 à 10000 kPa.

10. Un procédé selon l'une quelconque des revendications 1 à 6, dans lequel la charge contient des oléfines en $C_6$ à $C_{20}$ et est convertie en hydrocarbures bouillant dans l'intervalle distillats lourds-lubrifiants à une température de 140 à 285°C.

11